# EUROPEAN PATENT SPECIFICATION

(11) **EP 4 154 811 B1**
(45) Date of publication and mention of the grant of the patent: **23.10.2024**
(21) Application number: 21198152.7
(22) Date of filing: 22.09.2021
(51) Int. Cl.: A61B 5/11, A61B 5/103

(54) **GAIT EVALUATING SYSTEM**
GANGBEURTEILUNGSSYSTEM
SYSTÈME D'ÉVALUATION DE LA DÉMARCHE

(43) Date of publication of application: 29.03.2023
(73) Proprietor: Industrial Technology Research Institute, 310401 Hsinchu (TW)
(72) Inventor: HU, Je-Ping, 300 Hsinchu City (TW); LIN, Keng-Hsun, 701 Tainan City (TW); YANG MAO, Shih-Fang, 302 Zhubei City (TW); LI, Pin-Chou, 300 Hsinchu City (TW); WU, Jian-Hong, 542 Caotun Township, Nantou County (TW); LI, Szu-Ju, 500 Changhua City (TW); CHO, Hui-Yu, 433 Taichung City (TW); CHEN, Yu-Chang, 251 New Taipei City (TW); LU, Yen-Nien, 704 Tainan City (TW); HSU, Jyun-Siang, 114 Taipei City (TW); LEE, Nien-Ya, 552 Jiji Township, Nantou County (TW); HO, Kuan-Ting, 100 Taipei City (TW); TSAI, Ming-Chieh, 305 Xinpu Township, Hsinchu County (TW); HUANG, Ching-Yu, 330 Taoyuan City (TW)
(74) Representative: Becker, Eberhard

(56) References cited:
- WO-A1-2019/175899
- US-A1- 2012 253 234
- US-A1- 2014 114 214
- US-A1- 2015 260 514
- US-A1- 2017 087 416
- US-A1- 2020 000 373
- US-A1- 2021 161 430
- US-A1- 2021 204 836

## Description

### Technical Field

The invention relates to a human body evaluating technology, and in particularly to a gait evaluating system.

### Description of Related Art

With trends of decline of birth rate and/or increase of life expectancy, many countries in the world have entered a (super-)aging society. Among the care issues related to the elderly population, how to prevent the elderly population from falls has become one of the important issues.

After research, it is currently known that, gait-related parameters in people's walk may be used to predict future falls. For example, a normalized stride length of certain person may be used to predict the occurrence of repeated fall of the person in the next 6 or 12 months. Besides, people who walk relatively slowly also have a higher mortality rate. In addition, as people age, a forward inclination angle of the torso may also gradually increase. Moreover, for those suffering neurological diseases (e.g., Parkinson's disease, Alzheimer's disease, etc.), the angle of the torso may also be inclined forward or sideways.

Therefore, for those skilled in the art, if a mechanism can be designed where the gaits of people can be analyzed to determine whether the gaits of people are normal, it should be able to facilitates grasping the health condition of people, thus achieving the effect of preventing falls.

US 2021/204836 A1 discloses a wearable gait detection device, a walking ability improvement system and a wearable gait detection system. A load measurement part measures a load of a sole of the wearer's feet, a foot movement detection part at the shoes detects acceleration or angular velocity during feet movement, a centroid position calculation part calculates a centroid position of the feet based on changes in measured load, a movement locus calculation part calculates a movement locus of the feet based on acceleration or angular velocity detected by the foot movement detection part, a manifestation recognition part recognizes manifestation in the brain according to a specificity of centroid fluctuation of the feet based on the calculated centroid position and movement locus of the feet, and a sensory output part feeds back a sensation to the wearer based on a recognition result.
US 2021/161430 A1 discloses methods and systems for diagnosing, monitoring and treating persons at risk for falling. People are diagnosed before they actually start falling. The diagnosis includes trying out and identifying one or more fall triggers using virtual reality tools. Treatment includes training the persons using situations or triggers which are determined to be relevant for that person.
WO 2019/175899 A1 discloses a wearable device for gait analysis for screening gait and analysis of lower limb joint kinematics and kinetics including ankle, calf, knee, thigh, hip, pelvic, foot plantar pressure, clearance parameters and all spatial temporal parameters.
US 2020/000373 A1 discloses a quantitative gait training or analysis system employing instrumented footwear and an independent processing module. The instrumented footwear has sensors that permit the extraction of gait kinematics in real time and provide feedback from it.
US 2015/260514 A1 discloses a method for determining physical properties of ground stepped upon by a user wearing a footwear incorporating an accelerometer, and includes receiving a raw signal from the accelerometer; identifying, in the received raw signal, at least one characteristic signature; associating the at least one characteristic signature to physical properties of the ground; and generating a signal indicating the physical properties based on said association.
US 2017/087416 A1 discloses systems and methods for rehabilitation of limb motion including a thermal imaging device for imaging a subject or a plurality of sensors for sensing force or pressure exerted by a portion of the subject's body; a processor and a memory operably coupled to the processor. The processor is configured to receive force or pressure data measured by the sensors or image data captured by the thermal imaging device.
US 2012/253234 A1 discloses a system for analyzing gait using textile sensors including a sock sensing system, which comprises a sock and at least one switch, tension sensor, or pressure sensor for sensing a posture or movement; and a processor configured to receive signals from the sock sensing system and to analyze a gait parameter.
US 2014/114214 A1 discloses a system for measuring variation in the gait of a subject including a sensor arranged to measure variations in vertical position of the subject, a processor, and a display. The processor is arranged to identify a plurality of points in a first one of multiple steps and a plurality of points in a second one of the steps, to identify a plurality of pairs of the points, each pair comprising one point in each of the steps, to determine a value of height for each of the points in each of the pairs, and to control the display to produce a display plotting the heights of the two points in each pair against each other.

### SUMMARY

In view of the above, the invention provides a gait evaluating system, which may be used to solve the above technical problems.

The invention is provided by the appended claims. The following disclosure serves a better understanding of the present invention. Accordingly, the disclosure provides a gait evaluating system. The gait evaluating system includes a gait evaluating device configured to: obtain, from a pressure detection device, a plurality of pressure values of a user walking on the pressure detection device, where the pressure values correspond to a plurality of steps of the user; obtain a plurality of step feature values of the user based on the pressure values; obtain a plurality of walking limb feature values when the user walks on the pressure detection device based on a sensing data provided by a limb sensing device; and evaluate a gait of the user based on the step feature values and the walking limb feature values.

### BRIEF DESCRIPTION OF THE DRAWINGS

FIG. 1 is a schematic diagram illustrating a gait evaluating system according to an embodiment of the invention.
FIG. 2A is a schematic diagram illustrating a gait evaluating system according to a first embodiment of the invention.
FIG. 2B is a schematic diagram illustrating another gait evaluating system according to FIG. 2A.
FIG. 3 is a schematic diagram illustrating screening of an integrated skeleton diagram according to the first embodiment of the invention.
FIG. 4 is a schematic diagram illustrating a pressure detection device according to a second embodiment of the invention.
FIG. 5 is a flowchart illustrating a gait evaluating method according to an embodiment of the invention.
FIG. 6 is a schematic diagram illustrating a plurality of step feature values according to an embodiment of the invention.
FIG. 7 is a schematic diagram illustrating a plurality of reference bases for determining a first specific value according to an embodiment of the invention.

### DESCRIPTION OF THE EMBODIMENTS

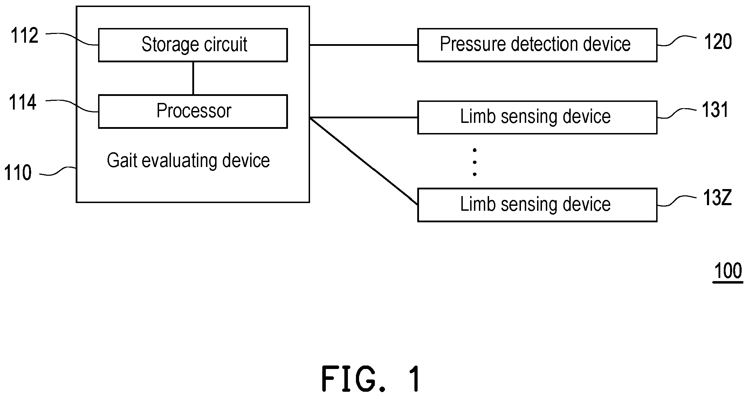

With reference to FIG. 1, which is a schematic diagram illustrating a gait evaluating system according to an embodiment of the invention. In FIG. 1, a gait evaluating system 100 may include a gait evaluating device 110, a pressure detection device 120, and limb sensing devices 131 to 13Z (where Z is a positive integer). In different embodiments, the gait evaluating device 110 is, for example but not limited to, various computer devices and/or smart devices.

As shown in FIG. 1, the gait evaluating device 110 may include a storage circuit 112 and a processor 114. The storage circuit 112 is, for example, any form of fixed or mobile random access memory (RAM), read-only memory (ROM), flash memory, hard drives, or other similar devices or a combination of these devices, and may be used to record a plurality of programming codes or modules.

The processor 114 is coupled to the storage circuit 112, and may be a general purpose processor, a special purpose processor, a conventional processor, a digital signal processor, a plurality of microprocessors, one or more microprocessors combined with a digital signal processor core, a controller, a microcontroller, an application specific integrated circuit (ASIC), a field programmable gate array (FPGA), any other type of integrated circuits, state machines, processors based on the Advanced RISC Machine (ARM), and the like.

In different embodiments, the pressure detection device 120 may be embodied as a pressure detection mat including a plurality of pressure detectors, and may also be used for a user (e.g., a person to be performed with gait evaluation) to walk on, to detect a distribution/value of pressure applied to the pressure detection device 120 at each step of the user.

In some embodiments, the limb sensing devices 131 to 13Z may each be embodied as a video camera to capture a walking image of the user walking on the pressure detection device 120.

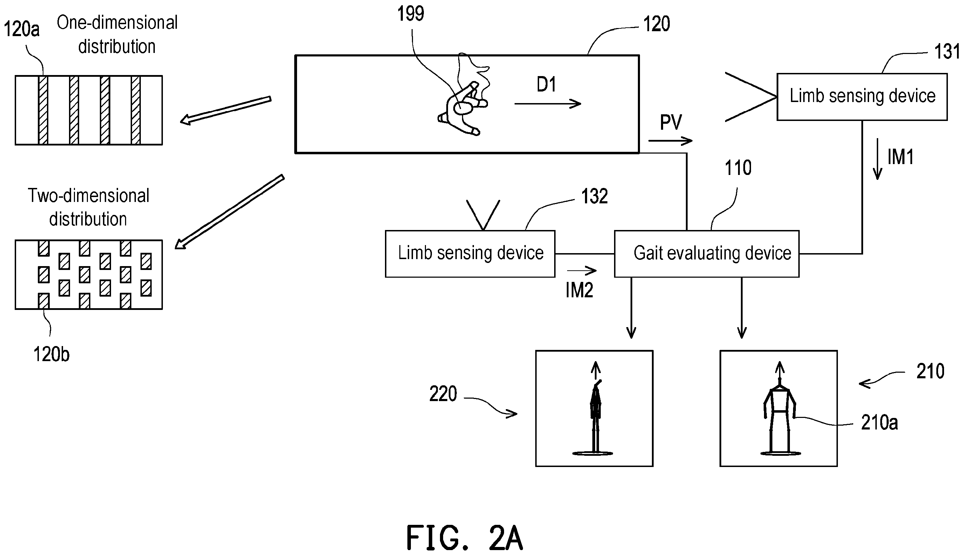

Reference may be to FIG. 2A, which is a schematic diagram illustrating a gait evaluating system according to a first embodiment of the invention. In FIG. 2A, the pressure detection device 120 may be embodied as a pressure detection mat, and a user 199 may walk on the pressure detection device 120 in a walking direction D1 upon request.

In an embodiment, the pressure detection device 120 may include a plurality of pressure detectors 120a exhibiting a one-dimensional distribution. In another embodiment, the pressure detection device 120 may also include a plurality of pressure detectors 120b exhibiting a two-dimensional distribution. Nonetheless, the disclosure is not limited thereto. In some embodiments, the length of the pressure detection mat may be greater than or equal to 3 meters, and the width may be greater than or equal to 0.4 meters. Besides, in some embodiments, the pressure detection mat may be provided with one pressure detector 120a (or one pressure detector 120b) per 50 cm² (or less). In some embodiments, the pressure detection mat may also be provided with one pressure detector 120a (or one pressure detector 120b) per 6.25 cm², but it is not limited thereto.

In the first embodiment, when the user 199 walks on the pressure detection device 120, the pressure detectors distributed on the pressure detection device 120 may detect a plurality of pressure values PV corresponding to steps of the user 199. The pressure detection device 120 may provide the pressure values PV to the gait evaluating device 110 for further analysis by the gait evaluating device 110.

In the first embodiment, the limb sensing devices 131 and 132 may be respectively embodied as a first video camera and a second video camera. The first video camera may be used to capture a first walking image IM1 when the user 199 walks on the pressure detection device 120, and the second video camera may be used to capture a second walking image IM2 when the user 199 walks on the pressure detection device 120.

As shown in FIG. 2A, the imaging direction of the limb sensing device 131 (i.e., the first video camera) may be opposite to the walking direction D1 of the user 199, to thereby capture a front image of the user 199 when walking. In addition, the imaging direction of the limb sensing device 132 (i.e., the second video camera) may be perpendicular to the walking direction D1 of the user 199, to thereby capture a side image (e.g., from the right side) of the user 199 when walking.

In the first embodiment, for the first walking image IM1 and the second walking image IM2 obtained by the first video camera and the second video camera at a t-th time point (where t is a time index value), the gait evaluating device 110 may obtain a first skeleton diagram 210 and a second skeleton diagram 220 respectively in the first walking image IM1 and the second walking image IM2. In the embodiment of the invention, the gait evaluating device 110 may obtain the first skeleton diagram 210 and the second skeleton diagram 220 respectively in the first walking image IM1 and the second walking image IM2 based on any known image processing algorithms, for example but not limited to, the literature document "Z. Cao, G. Hidalgo, T. Simon, S. -E. Wei and Y. Sheikh, OpenPose: Realtime Multi-Person 2D Pose Estimation Using Part Affinity Fields, in IEEE Transactions on Pattern Analysis and Machine Intelligence, vol. 43, no. 1, pp. 172-186, 1 Jan. 2021".

In the first embodiment, the first skeleton diagram 210 and the second skeleton diagram 220 may, for example, correspond to the human body posture of the user 199 at the t-th time point, and may each include a plurality of reference points corresponding to a plurality of joints of the user 199 (e.g., corresponds to a reference point 210a at a wrist of the user 199).

The gait evaluating device 110 projects the first skeleton diagram 210 and the second skeleton diagram 220 into a first integrated skeleton diagram based on the relative position between the first video camera and the second video camera. For related projection technology, reference may be made to the literature document "Z. Cao, G. Hidalgo, T. Simon, S. -E. Wei and Y. Sheikh, OpenPose: Realtime Multi-Person 2D Pose Estimation Using Part Affinity Fields, in IEEE Transactions on Pattern Analysis and Machine Intelligence, vol. 43, no. 1, pp. 172-186, 1 Jan. 2021".

In an embodiment, the first integrated skeleton diagram may include a plurality of joint angles (e.g., neck angle, shoulder angle, elbow angle, wrist angle, hip angle, knee angle, ankle angle, etc.) at the t-th time point. The joint angles correspond to the joints (e.g., neck, shoulders, elbows, wrists, hips, knees, ankles, etc.) of the user 199. After that, the gait evaluating device 110 may obtain a plurality of angle values of the joint angles, and take the angle values as a plurality of walking limb feature values of the user 199 at the t-th time point.

In some embodiments, after obtaining the first skeleton diagram 210, the second skeleton diagram 220, and/or the first integrated skeleton diagram, the gait evaluating device 110 may, for example, remove outliers from the skeleton diagrams based on the median filter or other similar noise reduction technology, and then remove high-frequency fluctuations from the skeleton diagrams through a fast Fourier transform (FFT). After that, the gait evaluating device 110 may also smooth the movement between the skeleton diagrams at different time points through polyfitting. Nonetheless, the disclosure is not limited thereto.

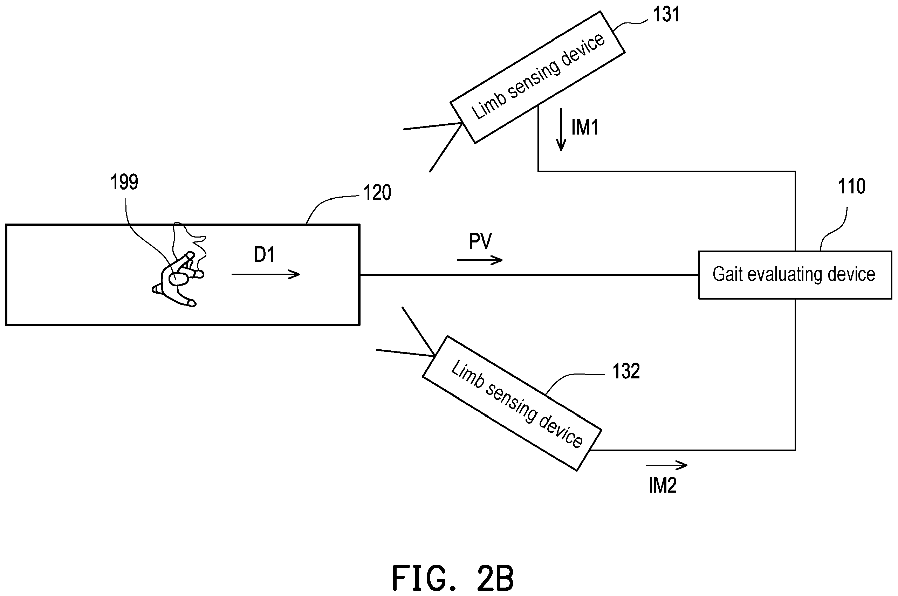

With reference to FIG. 2B, which is a schematic diagram illustrating another gait evaluating system according to FIG. 2A. In FIG. 2B, except that the imaging directions of the limb sensing devices 131 and 132 are different from those of FIG. 2A, the rest of the configuration is generally the same as that of FIG. 2A.

Specifically, in FIG. 2B, from two sides in front of the user 199, the limb sensing device 131 (i.e., the first video camera) and the limb sensing device 132 (i.e., the second video camera) may respectively capture the first walking image IM 1 and the second walking image IM2 of the user 199 when the user 199 walks on the pressure detection device 120 along the walking direction D1. After that, the gait evaluating device 110 may also obtain the first skeleton diagram 210 and the second skeleton diagram 220 respectively from the first walking image IM1 and the second walking image IM2, and project the first skeleton diagram 210 and the second skeleton diagram 220 into the first integrated skeleton diagram based on the aforementioned teaching.

In an embodiment, when human bodies other than that of the user 199 are present in the first walking image IM1 and the second walking image IM2, the gait evaluating device 110 may thus be unable to correctly obtain the integrated skeleton diagram corresponding to the user 199. Therefore, in the embodiments of the invention, human bodies other than that of the user 199 may be excluded through a specific mechanism, thereby increasing the gait evaluation accuracy.

According to the invention, after obtaining the first integrated skeleton diagram, the gait evaluating device 110 further determines whether the first integrated skeleton diagram satisfies a specified condition. If so, the gait evaluating device 110 then obtains the angle values of the joint angles, and take the angle values as the walking limb feature values of the user 199 at the t-th time point.

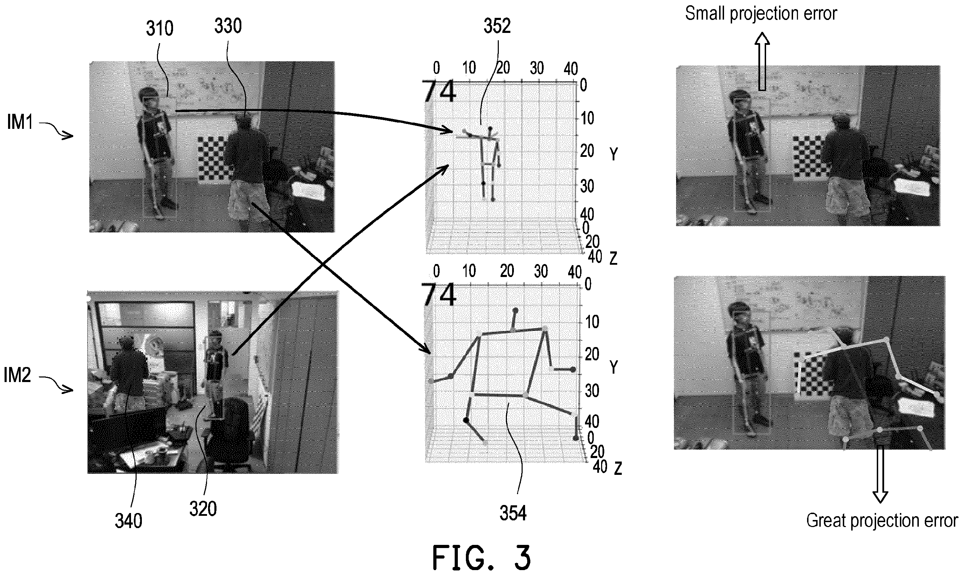

In an embodiment, the gait evaluating device 110 may determine whether the first walking image IM1 and the second walking image IM2 do not include skeleton diagrams corresponding to other human bodies. If so, this means that the first skeleton diagram 210 and the second skeleton diagram 220 correspond to the human body (i.e., the user 199) to be performed with gait evaluation at present. Therefore, the gait evaluating device 110 may correspondingly determine that the first integrated skeleton diagram satisfies the specified condition. If not, this means that skeleton diagrams corresponding to other human bodies are present in the first walking image IM1 and the second walking image IM2. Therefore, the gait evaluating device 110 may perform further screening to find the integrated skeleton diagram actually corresponding to the user 199. The related details accompanied with FIG. 3 will be further described.

With reference to FIG. 3, which is a schematic diagram illustrating screening of an integrated skeleton diagram according to the first embodiment of the invention. In this embodiment, it is assumed that the first walking image IM1 and the second walking image IM2 obtained at the t-th time point are as shown in FIG. 3.

From FIG. 3, it can be seen that the first walking image IM1 includes a first skeleton diagram 310 and a third skeleton diagram 330, and the second walking image IM2 includes a second skeleton diagram 320 and a fourth skeleton diagram 340. The first skeleton diagram 310 and the second skeleton diagram 320 correspond to the user to be performed with gait evaluation at present, and the third skeleton diagram 330 and the fourth skeleton diagram 340 correspond to another human body.

In this case, the gait evaluating device 110 may project the first skeleton diagram 310 and the second skeleton diagram 320 into a first integrated skeleton diagram 352, and project the third skeleton diagram 330 and the fourth skeleton diagram 340 into a second integrated skeleton diagram 354.

Then, the gait evaluating device 110 may obtain a first projection error of the first integrated skeleton diagram 352 and a second projection error of the second integrated skeleton diagram 354, and determine whether the first projection error is less than the second projection error.

In the scenario of FIG. 3, assuming that the first projection error is determined to be less than the second projection error, the gait evaluating device 110 may determine that the first integrated skeleton diagram 352 satisfies the specified condition, and may obtain the angle values of the joint angles in the first integrated skeleton diagram 352. After that, the gait evaluating device 110 may then take the angle values as the walking limb feature values of the user 199 at the t-th time point.

In other embodiments, in response to determining that the first projection error is not less than the second projection error, this means that the first integrated skeleton diagram 352 does not correspond to the human body to be performed with gait evaluation. Therefore, the gait evaluating device 110 may determine that the first integrated skeleton diagram 352 does not satisfy the specified condition. After that, the gait evaluating device 110 may obtain the walking limb feature values of the user 199 at the t-th time point based on the second integrated skeleton diagram 354.

Accordingly, even in a case where the gait evaluating system 100 of the first embodiment is disposed in a general field not dedicated to gait detection, in the embodiments of the invention, the target to be performed with gait evaluation may still be evaluated after other irrelevant human bodies are excluded.. Accordingly, an effect that the target may be evaluated without noticing that the target is being evaluated can be achieved.

In other embodiments, the gait evaluating system 100 in FIG. 2A and FIG. 2B may also include more video cameras to capture images of the user 199 from different angles. In this case, the gait evaluating device 110 may correspondingly obtain a more accurate integrated skeleton diagram, but it is not limited thereto.

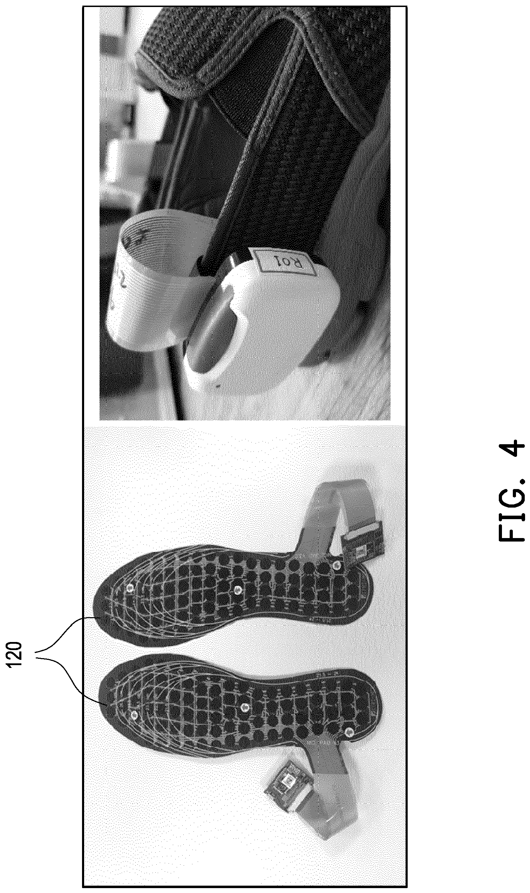

With reference to FIG. 4, which is a schematic diagram illustrating a pressure detection device according to a second embodiment of the invention. In FIG. 4, the pressure detection device 120 may be embodied as a pressure detection insole including a plurality of pressure detectors. In an embodiment, the pressure detection device 120 may be disposed in the shoes of the user 199 for the user 199 to wear and walk in. In this case, the pressure detection insole may detect the pressure value PV of each step of the user 199 when the user 199 walks, and may provide the pressure value PV corresponding to each step to the gait evaluating device 110. In the second embodiment, for the relevant measurement means, reference may be made to the content of the literature document "S. J. M. Bamberg, A. Y. Benbasat, D. M. Scarborough, D. E. Krebs and J. A. Paradiso, "Gait Analysis Using a Shoe-Integrated Wireless Sensor System," in IEEE Transactions on Information Technology in Biomedicine, vol. 12, no. 4, pp. 413-423, July 2008", which will not be repeatedly described herein.

In a third embodiment, the limb sensing devices 131 to 13Z may also be embodied as a plurality of dynamic capturing elements (e.g., inertial measurement units) that may be worn on the user 199. The dynamic capturing elements may be distributed at the joints (e.g., neck, shoulders, elbows, wrists, hips, knees, ankles, etc.) of the user 199 to capture movements of the joints.

For example, the gait evaluating device 110 may obtain, at the t-th time point, a plurality of three-dimensional spatial positions of the dynamic capturing elements, and accordingly establish a spatial distribution diagram of the dynamic capturing elements at the t-th time point. The spatial distribution diagram at the t-th time point may include a plurality of reference points corresponding to the dynamic capturing elements.

After that, according to the relative position between the joints of the user 199, the gait evaluating device 110 may connect the reference points in the spatial distribution diagram into the skeleton diagram (which may have an aspect similar to that of the first integrated skeleton diagram 352 of FIG. 3) of the user 199 at the t-th time point. The skeleton diagram may include the joint angles of the joints at the t-th time point. Then, the gait evaluating device 110 may obtain the angle values of the joint angles, and take the angle values as the walking limb feature values of the user 199 at the t-th time point.

In the third embodiment, for the details of detection through the dynamic capturing elements, reference may be made to the content of the literature documents "Schlachetzki JCM, Barth J, Marxreiter F, Gossler J, Kohl Z, Reinfelder S, Gassner H, Aminian K, Eskofier BM, Winkler J, Klucken J. Wearable sensors objectively measure gait parameters in Parkinson's disease. PLoS One. 2017 Oct 11" and "Qilong Yuan, I. Chen and Ang Wei Sin, "Method to calibrate the skeleton model using orientation sensors," 2013 IEEE International Conference on Robotics and Automation, 2013", which will not be repeatedly described herein.

In an embodiment, each joint of the user 199 may be predetermined with a corresponding angle range of motion. After obtaining the skeleton diagram of the user 199 at the t-th time point, the gait evaluating device 110 may determine whether the angle value of any joint angle in the skeleton diagram does not fall within the corresponding angle range of motion. If so, this means that the current skeleton diagram may contain a detection error, so the gait evaluating device 110 may correspondingly discard the skeleton diagram at the t-th time point.

For example, assuming that the angle range of motion corresponding to the elbow joint is 30 degrees to 180 degrees. In this case, if the gait evaluating device 110 determines that the joint angle of the elbow in the skeleton diagram at the t-th time point is less than 30 degrees or greater than 180 degrees, the gait evaluating device 110 may correspondingly discard the skeleton diagram at the t-th time point, but it is not limited thereto.

In the embodiments of the invention, the processor 114 may access the modules and programming codes recorded in the storage circuit 112 to realize the gait evaluating method provided by the invention, which will be described in detail as follows.

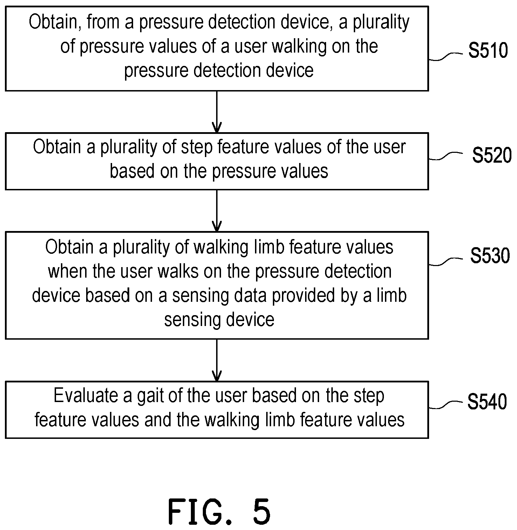

With reference to FIG. 5, which is a flowchart illustrating a gait evaluating method according to an embodiment of the invention. The method of the embodiment may be performed by the gait evaluating system 100 of FIG. 1. Each of steps of FIG. 5 accompanied with the elements shown in FIG. 1 will be described in detail below.

First, in step S510, the processor 114 may obtain, from the pressure detection device 120, a plurality of pressure values PV of the user 199 walking on the pressure detection device 120. In different embodiments, the processor 114 may obtain the pressure values PV with reference to the description in the above embodiments, which will not be repeated herein.

In step S520, the processor 114 may obtain a plurality of step feature values of the user 199 based on the pressure values PV. In different embodiments, based on the pressure values PV, the processor 114 may obtain at least one of a gait speed, a step length, a stride length, a cadence, a step width, a gait cycle, a stance time, a swing time, a center of pressure, a moving trajectory, a double support time, and a foot pressure distribution of the user 199 as the step feature values.

In some embodiments, the processor 114 may also obtain a stride-to-stride variation of the user 199 based on the pressure values PV. The stride-to-stride variation may include, but is not limited to, at least one of a swing time variation, a double support time variation, a step length time variation, and a stride length time variation.

In some embodiments, the user 199 may perform a timed up and go test (TUG) on the pressure detection device 120 upon request. In this case, based on the pressure values PV, the processor 114 may also obtain at least one of a get-up time, a turn time, a sit-down time, a walk speed, a walk time, and a total performance time of the user 199 in the timed up and go test as part of the step feature values. Nonetheless, the disclosure is not limited thereto.

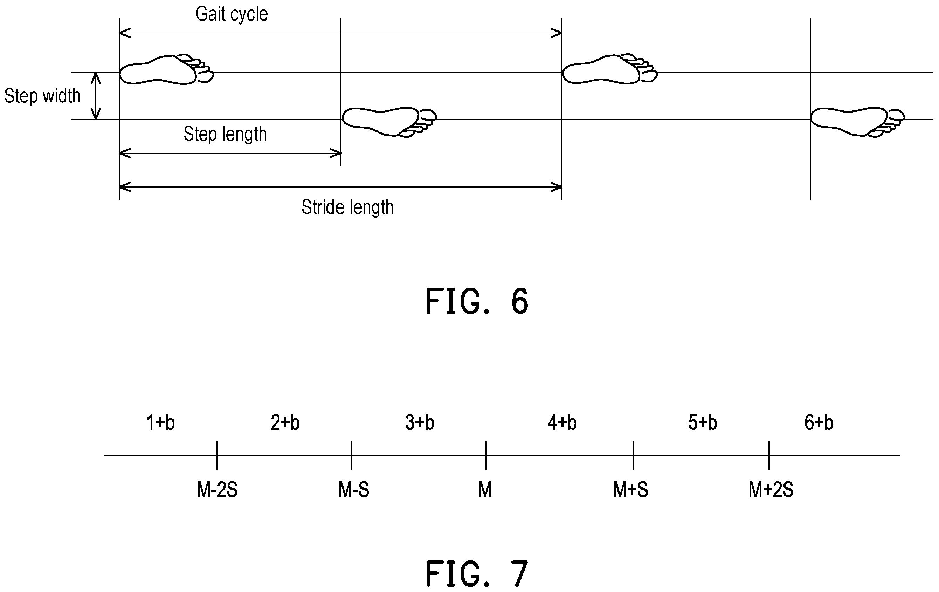

With reference to FIG. 6, which is a schematic diagram illustrating a plurality of step feature values according to an embodiment of the invention. FIG. 6 illustrates the difference between the terms such as step length, stride length, step width, and the like. For further details of the step feature values, reference may be made to the literature documents "Pirker W, Katzenschlager R. Gait disorders in adults and the elderly: A clinical guide. Wien Klin Wochenschr. 2017;129 (3-4):81-95. doi: 10.1007/s00508-016-1096-4" and "Bohannon RW, Williams Andrews A. Normal walking speed: a descriptive meta-analysis. Physiotherapy. 2011", which will not be repeatedly described herein.

Besides, for the details of obtaining the step feature values based on the pressure values PV, reference may be made to the literature documents "Yoo SD, Kim HS, Lee JH, Yun DH, Kim DH, Chon J, Lee SA, Han YJ, Soh YS, Kim Y, Han S, Lee W, Han YR. Biomechanical Parameters in Plantar Fasciitis Measured by Gait Analysis System With Pressure Sensor. Ann Rehabil Med. 2017 Dec" and "Greene BR, O'Donovan A, Romero-Ortuno R, Cogan L, Scanaill CN, Kenny RA. Quantitative falls risk assessment using the timed up and go test. IEEE Trans Biomed Eng. 2010 Dec", which will not be repeatedly described herein.

In step S530, based on sensing data provided by the limb sensing devices 131 to 13Z, the processor 114 may obtain a plurality of walking limb feature values when the user 199 walks on the pressure detection device. In different embodiments, the processor 114 may obtain the walking limb feature values (e.g., a plurality of angle values of a plurality of joint angles of the user 199) based on the sensing data (e.g., the first walking image IM1 and the second walking image IM2) provided by the limb sensing devices 131 to 13Z with reference to the description in the above embodiments, which will not be repeated herein.

Then, in step S540, the processor 114 may evaluate a gait of the user 199 based on the step feature values and the walking limb feature values. In different embodiments, the processor 114 may evaluate the gait of the user 199 based on different ways, which will be further described below.

In a fourth embodiment, the processor 114 may determine whether the step feature values and the walking limb feature values of the user 199 do not satisfy a corresponding first statistical standard. In response to determining that Y of the step feature values and the walking limb feature values of the user 199 (where Y is a specified number) does not satisfy the corresponding first statistical standard, the processor 114 may determine that the gait of the user 199 belongs to an abnormal gait, and in the opposite case, the processor 114 may determine that the gait of the user 199 belongs to a normal gait.

In different embodiments, the first statistical standard corresponding to the step feature values and the walking limb feature values may be determined in different ways.

For example, an average gait speed of males in the sixties is statistically 1.34m/s. Accordingly, when the user 199 is a male between 60 and 69 years old, the first statistical standard corresponding to the gait speed may be set to 1.34m/s. Besides, since an average gait speed of healthy elder people is statistically 1.1m/s to 1.5m/s, when the user 199 is an elder person, the first statistical standard corresponding to the gait speed may be set to 1.1m/s. Nonetheless, the disclosure is not limited thereto.

In an embodiment, the normal stride length of ordinary people is about 76 to 92cm on average, so the first statistical standard corresponding to the stride length of the user 199 may be set to 76cm. Nonetheless, the disclosure is not limited thereto.

Based on a similar concept to the above teaching, the processor 114 may also correspondingly determine the first statistical standard corresponding to the step feature values and the walking limb feature values, for example, the cadence, a TUG time, a torso inclination angle, the stride-to-stride variation, a heel strike angle, and a toe-off angle based on the relevant literature documents/statistical data (e.g., the content of "Gong H, Sun L, Yang R, Pang J, Chen B, Qi R, Gu X, Zhang Y, Zhang TM. Changes of upright body posture in the sagittal plane of men and women occurring with aging - a cross sectional study. BMC Geriatr. 2019 Mar 5", "Oeda T, Umemura A, Tomita S, Hayashi R, Kohsaka M, Sawada H. Clinical factors associated with abnormal postures in Parkinson's disease. PLoS One. 2013 Sep 19", and "Schlachetzki JCM, Barth J, Marxreiter F, Gossler J, Kohl Z, Reinfelder S, Gassner H, Aminian K, Eskofier BM, Winkler J, Klucken J. Wearable sensors objectively measure gait parameters in Parkinson's disease. PLoS One. 2017 Oct 11").

For example, the first statistical standard corresponding to the cadence may be 1.2 times/s, and the first statistical standard corresponding to the TUG time may be less than 20 seconds. In addition, the first statistical standard of the torso inclination angle is, for example, that a square root of the sum of squares of the total inclination angles toward the front and back/the left and right must be less than 10 degrees. The first statistical standard of the stride-to-stride variation is, for example, that the step length time variation must be less than 4%, the swing time variation must be less than 5%, the double support time variation must be less than 8%, the stride length time variation must be less than 4%, and the like. Nonetheless, the disclosure is not limited thereto.

Besides, the first statistical standard of the heel strike angle, for example, must be greater than 20 degrees, and the first statistical standard of the toe-off angle, for example, must be greater than 55 degrees. Nonetheless, the disclosure is not limited thereto.

In an embodiment, when the user 199 belongs to a specific group including a plurality of group members, the processor 114 may also determine the first statistical standard corresponding to each step feature value and each walking limb feature value based on the properties of the specific group.

For example, the processor 114 may obtain a plurality of reference step feature values and a plurality of reference walking limb feature values of the group members of the specific group, and accordingly estimate the first statistical standard of each of the step feature values and each of the walking limb feature values. In some embodiments, the reference step feature values and the reference walking limb feature values of each group member may correspond to the step feature values and the walking limb feature values of the user A.

For example, when obtaining the first statistical standard corresponding to the stride length, the processor 114 may obtain the stride length of each group member, and then take the first 90% of the stride lengths of the group members as the first statistical standard of the stride length. In this case, when the stride length of the user 199 falls within the last 10% of the specific group, the processor 114 may then determine that the stride length of the user 199 does not satisfy the corresponding first statistical standard. For other step feature values and other walking limb feature values, the processor 114 may determine the corresponding first statistical standard based on a similar principle, the details of which will not be repeatedly described herein.

In an embodiment, the processor 114 may also determine the first statistical standard corresponding to each step feature value and each walking limb feature value based on previously measured historical step feature values and historical walking limb feature values of the user 199.

In an embodiment, the processor 114 may obtain the step feature values and the walking limb feature values of the user 199 measured in the previous test as the historical step feature values and the historical walking limb feature values of the user 199. After that, the processor 114 may determine the first statistical standard of each of the step feature values and each of the walking limb feature values of the user 199 based on a specific ratio of each of the historical step feature values and each of the historical walking limb feature values.

For example, when determining the first statistical standard of the stride length of the user 199, the processor 114 may obtain the previously measured stride length (hereinafter referred to as historical stride length) of the user 199, and take a specific ratio (e.g., 90%) of historical stride length as the first statistical standard of the stride length of the user 199. When the processor 114 determines that the stride length of the user 199 does not satisfy the corresponding first statistical standard (e.g., the stride length of the user 199 is less than 90% of the historical stride length), this means that the stride length of the user 199 has shown a certain extent of regression (e.g., regression by more than 10%), which may thus be used as a basis for determining that the gait of the user 199 is abnormal. For other step feature values and other walking limb feature values, the processor 114 may determine the corresponding first statistical standard based on a similar principle, the details of which will not be repeatedly described herein.

In different embodiments, the value of Y may be set by the designer depending on the needs. For example, in a case where Y is set to 1, the processor 114 may determine that the gait of the user 199 belongs to an abnormal gait when any one of the step feature values and the walking limb feature values of the user 199 does not satisfy the corresponding first statistical standard. Moreover, in a case where Y is set to 2, the processor 114 may determine that the gait of the user 199 belongs to an abnormal gait when any two of the step feature values and the walking limb feature values of the user 199 do not satisfy the corresponding first statistical standard. Nonetheless, the disclosure is not limited thereto.

In a fifth embodiment, the processor 114 may select an N number of specific values from the step feature values and the walking limb feature values of the user 199, and may map the specific values into a plurality of map values according to a K number of reference bases corresponding to each specific value, where N and K are positive integers, and each map value falls within a predetermined range.

After that, the processor 114 may perform a weighting operation on the map values to obtain a weighting operation result. Then, in response to determining that the weighting operation result does not satisfy a second statistical standard, the processor 114 may determine that the gait of the user 199 belongs to an abnormal gait, and in the opposite case, the processor 114 may determine that the gait of the user 199 belongs to a normal gait. Nonetheless, the disclosure is not limited thereto.

In an embodiment, for a first specific value in the specific values, the processor 114 may obtain a reference mean and a reference difference factor corresponding to the first specific value, accordingly estimate the reference bases corresponding to the first specific value.

In an embodiment, the reference mean may be represented as M, and the reference difference factor may be represented as S. In an embodiment, the reference bases corresponding to the first specific value may be represented as M+iS, where i is an integer, *i* ∈ [-*a*, ... , +*a*], and a is a positive integer.

With reference to FIG. 7, which is a schematic diagram illustrating a plurality of reference bases for determining a first specific value according to an embodiment of the invention. In FIG. 7, assuming that a is 2, then the reference bases may respectively be M-2S, M-S, M, M+S, and M+2S, but are not limited thereto.

Based on the architecture of FIG. 7, the processor 114 may map the first specific value into a first map value in the map values. In an embodiment, in response to determining that the first specific value is between the j-th reference basis and the j+ I-th reference basis, the processor 114 may determine that the first map value is j+1+b, where 1 ≤ *j* ≤ *K* - 1, and b is a constant. In response to determining that the first specific value is less than the first reference basis (e.g., M-2S), the processor 114 may determine that the first map value is 1+b. In response to determining that the first specific value is greater than the K-th reference basis (e.g., M+2S), the processor 114 may determine that the first map value is K + 1 +b.

For ease of description, it is assumed that b is 0 in the following, but the invention is not limited thereto. In this case, when the first specific value is less than the first reference basis (e.g., M-2S), the processor 114 may map the first specific value into 1. When the first specific value is between the first reference basis (i.e., M-2S) and the second reference basis (i.e., M-S), the processor 114 may map the first specific value into 2. When the first specific value is between the second reference basis (i.e., M-S) and the third reference basis (i.e., M), the processor 114 may map the first specific value into 3. When the first specific value is between the third reference basis (i.e., M) and the fourth reference basis (i.e., M+S), the processor 114 may map the first specific value into 4. When the first specific value is between the fourth reference basis (i.e., M+S) and the fifth reference basis (M+2S), the processor 114 may map the first specific value into 5. When the first specific value is greater than the fifth reference basis (e.g., M+2S), the processor 114 may map the first specific value into 6. Nonetheless, the disclosure is not limited thereto.

In the scenario of FIG. 7, it can be seen that the predetermined range of the first map value is, for example, 1+b, 2+b, 3+b, 4+b, 5+b, and 6+b. In other embodiments, for other specific values, the processor 114 may map each of the specific values into the corresponding map values based on the above teaching, and the map values may have the same predetermined range as that of the first map value. Nonetheless, the disclosure is not limited thereto.

In different embodiments, the processor 114 may determine the reference mean (i.e., M) and the reference difference factor (i.e., S) of the first specific value based on different principles.

For example, assuming that the gait speed is the first specific value under consideration, then the processor 114 may obtain a mean of the general normal gait speed as the reference mean of the first specific value, and then take the specific ratio of the mean as the reference difference factor based on the relevant literature documents (e.g., "Bohannon RW, Williams Andrews A. Normal walking speed: a descriptive meta-analysis. Physiotherapy. 2011 Sep" or "Studenski S, Perera S, Patel K, Rosano C, Faulkner K, Inzitari M, Brach J, Chandler J, Cawthon P, Connor EB, Nevitt M, Visser M, Kritchevsky S, Badinelli S, Harris T, Newman AB, Cauley J, Ferrucci L, Guralnik J. Gait speed and survival in older adults. JAMA. 2011 Jan 5"). For example, assuming that the specific ratio is 10%, then the reference bases corresponding to the gait speed may be, for example but not limited to, 80%, 90%, 100%, 110%, and 120% of M.

For another example, assuming that the forward torso inclination angle is the first specific value under consideration, then the processor 114 may obtain a mean of the general normal forward torso inclination angle as the reference mean of the first specific value, and then take the specific ratio of the mean as the reference difference factor based on the relevant literature documents (e.g., "Gong H, Sun L, Yang R, Pang J, Chen B, Qi R, Gu X, Zhang Y, Zhang TM. Changes of upright body posture in the sagittal plane of men and women occurring with aging - a cross sectional study. BMC Geriatr. 2019 Mar 5"). For example, assuming that the specific ratio is 10%, then the reference bases corresponding to the forward torso inclination angle may be, for example but not limited to, 80%, 90%, 100%, 110%, and 120% of M. For other first specific values, the processor 114 may determine the corresponding reference bases based on the above teaching, the details of which will not be repeatedly described herein.

In some embodiments, the processor 114 may also find a first reference value corresponding to the first specific value from the reference step feature values and the reference walking limb feature values of each group member in the specific group. After that, the processor 114 may then obtain a mean and a standard deviation of the first reference value of each group member, and define the mean and the standard deviation respectively as the reference mean (i.e., M) and the reference difference factor (i.e., S) of the first specific value.

For example, assuming that the first specific value is the stride length of the user 199, then the processor 114 may find the stride length of each group member as the first reference value of each group member, and accordingly estimate a mean and a standard deviation of the stride length of each group member. After that, the processor 114 may take the mean and the standard deviation as the reference mean (i.e., M) and the reference difference factor (i.e., S) of the first specific value, and accordingly determine the reference bases corresponding to the stride length.

For another example, assuming that the first specific value is the gait speed of the user 199, then the processor 114 may find the gait speed of each group member as the first reference value of each group member, and accordingly estimate a mean and a standard deviation of the gait speed of each group member. After that, the processor 114 may take the mean and the standard deviation as the reference mean (i.e., M) and the reference difference factor (i.e., S) of the first specific value, and accordingly determine the reference bases corresponding to the gait speed.

After obtaining an N number of map values of the N number of specific values, the processor 114 may perform the weighting operation on the map values to generate the weighting operation result. In an embodiment, the respective weights of the N number of map values may be determined by the designer depending on the needs. For example, assuming that the N number of specific values are the gait speed and the torso inclination angle of the user 199, then after mapping the gait speed and the torso inclination angle of the user 199 into two corresponding map values, the processor 114 may obtain the corresponding weighting operation result based on formula " *P*₁ × *W*₁ + *P*₂ × *W*₂ ", where *P*₁ and *P*₂ are the map values respectively corresponding to the gait speed and the torso inclination angle, and *W*₁ and *W*₂ are weights (both of which may be 50%, for example) respectively corresponding to *P*₁ and *P*₂. Nonetheless, the disclosure is not limited thereto.

After that, the processor 114 may determine whether the weighting operation result satisfies the second statistical standard. In some embodiments, the processor 114 may determine the second statistical standard based on a mechanism below.

For example, the processor 114 may obtain an N number of reference values corresponding to the N number of specific values from the reference step feature values and the reference walking feature values of each group member of the specific group. Following the above example, assuming that the gait speed and the torso inclination angle of the user 199 are the N number of specific values under consideration, then the processor 114 may obtain the gait speed and the torso inclination angle of each group member as the N number of reference values of each group member.

After that, the processor 114 may map the N number of reference values of each group member into a plurality of reference map values according to the reference bases corresponding to each specific value, where each reference map value falls within the predetermined range. In an embodiment, the processor 114 may map the N number of reference values of each group member into the corresponding reference map values with reference to mapping the first specific value of the user 199 into the corresponding first map value. Therefore, the details will not be repeatedly described herein.

Then, the processor 114 may perform a weighting operation on the N number of reference map values of each group member to generate a reference weighting operation result of each group member. Following the above example, after mapping the gait speed and the torso inclination angle of a certain group member into two corresponding reference map values, the processor 114 may obtain the corresponding reference weighting operation result based on formula " *P'*₁ × *W*₁ + *P'*₂ × *W*₂ ", where *P'*₁ and *P'*₂ are the reference map values respectively corresponding to the gait speed and the torso inclination angle of the certain group member.

After that, the processor 114 may determine the second statistical standard based on the reference weighting operation result of each group member. In an embodiment, the processor 114 may, for example, take the last 90% of the reference weighting operation results of the group members as the second statistical standard. In this case, in response to determining that the weighting operation result of the user 199 falls within the last 90% of the reference weighting operation results of the group member, the processor 114 may determine that the weighting operation result of the user 199 satisfies the second statistical standard. On the other hand, in response to determine that the weighting operation result of the user 199 falls within the top 10% of the reference weighting operation results of the group member, the processor 114 may determine that the weighting operation result of the user 199 does not satisfy the second statistical standard. Nonetheless, the disclosure is not limited thereto.

In an embodiment, in the case where it is determined that the gait of the user 199 belongs to an abnormal gait, the processor 114 may further determine whether the gait of the user 199 belongs to a non-neuropathic gait or a neuropathic gait.

In an embodiment, the processor 114 may determine whether the stride-to-stride variation of the user 199 satisfies a third statistical standard. If so, the processor 114 may determine that the gait of the user 199 belongs to a neuropathic gait, and in the opposite case, the processor 114 may determine that the gait of user belongs to a non-neuropathic gait.

In an embodiment, the processor 114 may determine the third statistical standard based on the stride-to-stride variation of each group member in the specific group. For example, the processor 114 may take the first 70% of the stride-to-stride variations of the group members as the third statistical standard. In this case, in response to determining that the stride-to-stride variation of the user 199 falls within the top 70% of the stride-to-stride variations of the group members, the processor 114 may determine that the stride-to-stride variation of the user 199 satisfies the third statistical standard. On the other hand, in response to determining that the stride-to-stride variation of the user 199 falls within the last 30% of the stride-to-stride variations of the group members, the processor 114 may determine that the stride-to-stride variation of the user 199 does not satisfy the third statistical standard. Nonetheless, the disclosure is not limited thereto.

In an embodiment, in response to determining that the gait of the user 199 belongs to an abnormal gait, the processor 114 may also provide a corresponding enablement suggestion.

For example, assuming that the gait of the user 199 is a non-neuropathic gait (e.g., gait abnormality resulting from bow legs, knock knees, or the like), the processor 114 may provide a strength training suggestion corresponding to the non-neuropathic gait as the enablement suggestion. In an embodiment, the strength training suggestion may base its content on the relevant literature documents of physical therapy (e.g., literature documents of strength training for treatment of bow legs or knock knees). Nonetheless, the disclosure is not limited thereto.

In addition, assuming that the gait of the user 199 belongs to a neuropathic gait (e.g., gait abnormality caused by Parkinson's disease or Alzheimer's disease), then the processor 114 may provide a rhythmic gait training suggestion corresponding to the neuropathic gait as the enablement suggestion. For the content of the rhythmic gait training suggestion, reference may be made to literature documents, for example but not limited to, "Pacchetti C., Mancini F., Aglieri R., Fundaro C., Martignoni E., Nappi G., Active musictherapy in Parkinson's disease: An integrative method for motor and emotionalrehabilitation. Psychosom Med 2000; 62(3): 386-93" and "deDreu MJ., van der Wilk AS., Poppe E., Kwakkel G., van Wegen EE., Rehabilitation, exercise therapy and music in patients with Parkinson's disease: A meta-analysis of the effects of music-based movement therapy on walking ability, balance and quality of life. Parkinsonism RelatDisord. 2012; 18 Suppl 1: S114-9".

In summary of the foregoing, in the invention, after the step feature values and the walking limb feature values when the user walks are obtained through the pressure detection device and the limb sensing device, these feature values may be integrated for evaluating the gait of the user. Accordingly, in the invention, after the user takes a small amount of walk, the health condition of the user can be grasped accordingly, allowing relevant caregivers to take corresponding measures based on the health condition of the user, thereby achieving the effect of preventing the user from falls.

## Claims

1. A gait evaluating device (110), comprising:
a storage circuit, storing a programming code;
a processor, coupled to the storage circuit and accessing the programming code to perform:
obtaining (S510), from a pressure detection device (120), a plurality of pressure values (PV) of a user (199) walking on the pressure detection device (120), wherein the pressure values (PV) correspond to a plurality of steps of the user (199);
obtaining (S520) a plurality of step feature values of the user (199) based on the pressure values (PV);
obtaining (S530) a plurality of walking limb feature values when the user (199) walks on the pressure detection device (120) based on a sensing data provided by at least one limb sensing device (131-13Z),
wherein
the at least one limb sensing device comprises at least a first video camera and a second video camera having different imaging ranges, and wherein
the processor is adapted to perform:
obtaining, at a t-th time point, a first walking image captured by the first video camera when the user walks on the pressure detection device, and obtaining a first skeleton diagram in the first walking image;
obtaining, at the t-th time point, a second walking image captured by the second video camera when the user walks on the pressure detection device, and obtaining a second skeleton diagram in the second walking image, wherein the first skeleton diagram and the second skeleton diagram correspond to a first human body;
projecting, based on a relative position between the first video camera and the second video camera, the first skeleton diagram and the second skeleton diagram into a first integrated skeleton diagram, the first integrated skeleton diagram comprising a plurality of joint angles at the t-th time point, wherein the joint angles correspond to a plurality of joints of the first human body; and
in response to determining that the first integrated skeleton diagram satisfies a specified condition, obtaining a plurality of angle values of the joint angles, and taking the angle values as the walking limb feature values of the user at the t-th time point; and
evaluating (S540) a gait of the user (199) based on the step feature values and the walking limb feature values.

2. The gait evaluating device (110) as described in claim 1, wherein c:
in response to determining that Y of the step feature values and the walking limb feature values of the user (199) do not satisfy a corresponding first statistical standard, determining that the gait of the user (199) belongs to an abnormal gait, where Y is a specified number.

3. The gait evaluating device (110) as described in claim 2, wherein the user (199) belongs to a specific group, and the method comprises:
obtaining a plurality of reference step feature values and a plurality of reference walking limb feature values of a plurality of group members of the specific group, and accordingly estimating the first statistical standard of each of the step feature values and each of the walking limb feature values.

4. The gait evaluating device (110) as described in claim 2, wherein the processor further performs:
obtaining a plurality of historical step feature values and a plurality of historical walking limb feature values of the user (199), wherein the historical step feature values and the historical walking limb feature values correspond to the step feature values and the walking limb feature values of the user (199); and
determining, based on a specific ratio of each of the historical step feature values and each of the historical walking limb feature values, the first statistical standard of each of the step feature values and each of the walking limb feature values.

5. The gait evaluating device (110) as described in claim 1, wherein the processor performs:
selecting an N number of specific values from the step feature values and the walking limb feature values, and mapping the specific values into a plurality of map values according to a K number of reference bases corresponding to the specific values, where N and K are positive integers, and each of the map values falls within a predetermined range;
performing a weighting operation on the map values to obtain a weighting operation result; and
in response to determining that the weighting operation result does not satisfy a second statistical standard, determining that the gait of the user (199) belongs to an abnormal gait.

6. The gait evaluating device (110) as described in claim 5, wherein the specific values comprise a first specific value, and the processor performs:
obtaining a reference mean and a reference difference factor corresponding to the first specific value, and accordingly estimating the reference bases corresponding to the first specific value.

7. The gait evaluating device (110) as described in claim 6, wherein the user (199) belongs to a specific group, the specific group comprises a plurality of group members, and each of the group members has a plurality of reference step feature values and a plurality of reference walking limb feature values, and the processor performs:
finding a first reference value corresponding to the first specific value from the reference step feature values and the reference walking limb feature values of each of the group members; and
obtaining a mean and a standard deviation of the first reference value of each of the group members, and respectively define the mean and the standard deviation as the reference mean and the reference difference factor of the first specific value.

8. The gait evaluating device (110) as described in claim 6, wherein the map values comprise a first map value corresponding to the first specific value, the reference mean is represented as M, the reference difference factor is represented as S, and the reference bases corresponding to the first specific value is represented as M+iS, where i is an integer, *i* ∈ [*-a, ... ,* +*a*], and a is a positive integer, and the processor performs:
in response to determining that the first specific value is between a j-th reference basis and a j+1-th reference basis in the reference bases, determining that the first map value is j+1+b, where 1 ≤ *j* ≤ *K* - 1, and b is a constant;
in response to determining that the first specific value is less than a first reference basis in the reference bases, determining that the first map value is 1+b; and
in response to determining that the first specific value is greater than a K-th reference basis in the reference bases, determining that the first map value is K+1+b.

9. The gait evaluating device (110) as described in claim 5, wherein the user (199) belongs to a specific group, the specific group comprises a plurality of group members, and each of the group members has a plurality of reference step feature values and a plurality of reference walking limb feature values, and the processor performs:
obtaining an N number of reference values corresponding to the specific values from the reference step feature values and the reference walking feature values of each of the group members;
mapping, according to the reference bases corresponding to each of the specific values, the reference values of each of the group members into a plurality of reference map values, wherein each of the reference map values falls within the predetermined range,
performing the weighting operation on the reference map values of each of the group members to generate a reference weighting operation result of each of the group members; and
determining, based on the reference weighting operation result of each of the group members, the second statistical standard.

10. The gait evaluating device (110) as described in claim 2 or claim 5, wherein the step feature values and the walking limb feature values comprise a stride-to-stride variation, and the processor performs:
in response to determining that the gait of the user (199) belongs to the abnormal gait, and the stride-to-stride variation satisfies a third statistical standard, determining that the gait of the user (199) belongs to a neuropathic gait; and
in response to determining that the gait of the user (199) does not belong to the abnormal gait, or the stride-to-stride variation does not satisfy the third statistical standard, determining that the gait of the user (199) belongs to a non-neuropathic gait.

11. The gait evaluating device (110) as described in claim 10, wherein the user (199) belongs to a specific group, the specific group comprises a plurality of group members, and each of the group members has the corresponding stride-to-stride variation, and the processor performs:
determining, based on the stride-to-stride variation of each of the group members, the third statistical standard.

12. The gait evaluating device (110) as described in claim 10, wherein in response to determining that the gait of the user (199) belongs to the non-neuropathic gait or the neuropathic gait, an enablement suggestion is provided.

13. The gait evaluating device (110) as described in claim 12, wherein in response to determining that the gait of the user (199) belongs to the non-neuropathic gait, a strength training suggestion corresponding to the non-neuropathic gait is provided as the enablement suggestion.

14. The gait evaluating device (110) as described in claim 12, wherein in response to determining that the gait of the user (199) belongs to the neuropathic gait, a rhythmic gait training suggestion corresponding to the neuropathic gait is provided as the enablement suggestion.

15. A gait evaluating system (100), comprising:
a pressure detection device (120);
at least one limb sensing device (131-13Z); and
a gait evaluating device (110) configured to:
obtain, from the pressure detection device (120), a plurality of pressure values (PV) of a user (199) walking on the pressure detection device (120), wherein the pressure values (PV) correspond to a plurality of steps of the user (199);
obtain a plurality of step feature values of the user (199) based on the pressure values (PV);
obtain a plurality of walking limb feature values when the user (199) walks on the pressure detection device (120) based on a sensing data provided by the at least one limb sensing device (131-13Z),
wherein
the at least one limb sensing device comprises at least a first video camera and a second video camera having different imaging ranges, and
wherein
the gait evaluating device (110) is adapted to perform:
obtaining, at a t-th time point, a first walking image captured by the first video camera when the user walks on the pressure detection device, and obtaining a first skeleton diagram in the first walking image;
obtaining, at the t-th time point, a second walking image captured by the second video camera when the user walks on the pressure detection device, and obtaining a second skeleton diagram in the second walking image, wherein the first skeleton diagram and the second skeleton diagram correspond to a first human body;
projecting, based on a relative position between the first video camera and the second video camera, the first skeleton diagram and the second skeleton diagram into a first integrated skeleton diagram, the first integrated skeleton diagram comprising a plurality of joint angles at the t-th time point, wherein the joint angles correspond to a plurality of joints of the first human body; and
in response to determining that the first integrated skeleton diagram satisfies a specified condition, obtaining a plurality of angle values of the joint angles, and taking the angle values as the walking limb feature values of the user at the t-th time point; and
evaluating a gait of the user (199) based on the step feature values and the walking limb feature values.

16. The system as described in claim 15, wherein the pressure detection device (120) comprises a pressure detection insole worn on a foot of the user (199), wherein the pressure detection insole detects the pressure values (PV) of the steps of the user (199); or
the pressure detection device (120) comprises a pressure detection mat distributed with a plurality of pressure detectors (120a, 120b), wherein the pressure detection mat detects the pressure values (PV) of the steps of the user (199) through the pressure detectors.

17. The system as described in claim 15, wherein the at least one limb sensing device (131-13Z) comprises a plurality of dynamic capturing elements worn on the user.

## Patentansprüche

1. Gangbewertungsvorrichtung (110), umfassend:
eine Speicherschaltung, die einen Programmiercode speichert;
einen Prozessor, der mit der Speicherschaltung verbunden ist und auf den Programmiercode zugreift, um durchzuführen:
Erhalten (S510) einer Vielzahl von Druckwerten (PV) eines Benutzers (199), der auf der Druckerfassungsvorrichtung (120) geht, von einer Druckerfassungsvorrichtung (120), wobei die Druckwerte (PV) einer Vielzahl von Schritten des Benutzers (199) entsprechen;
Erhalten (S520) einer Vielzahl von Schrittmerkmalswerten des Benutzers (199) basierend auf den Druckwerten (PV);
Erhalten (S530) einer Vielzahl von Geh-Gliedmaßen-Merkmalswerten, wenn der Benutzer (199) auf der Druckerfassungsvorrichtung (120) geht, basierend auf Erfassungsdaten, die von mindestens einer Gliedmaßen-Erfassungsvorrichtung (131-13Z) bereitgestellt werden, wobei die mindestens eine Gliedmaßen-Erfassungsvorrichtung mindestens eine erste Videokamera und eine zweite Videokamera mit unterschiedlichen Bildbereichen umfasst, und wobei der Prozessor angepasst ist, auszuführen:
Erhalten, zu einem t-ten Zeitpunkt, eines ersten Gehbildes, das von der ersten Videokamera aufgenommen wird, wenn der Benutzer auf der Druckerkennungsvorrichtung geht, und Erhalten eines ersten Skelettdiagramms in dem ersten Gehbild,
Erhalten, zu einem t-ten Zeitpunkt ein zweites Gehbild, das von der zweiten Videokamera aufgenommen wurde, wenn der Benutzer auf der Druckerfassungsvorrichtung geht, und
Erhalten eines zweiten Skelettdiagramms in dem zweiten Gehbild, wobei das erste Skelettdiagramm und das zweite Skelettdiagramm einem ersten menschlichen Körper entsprechen;
Projizieren, basierend auf einer relativen Position zwischen der ersten Videokamera und der zweiten Videokamera, des ersten Skelettdiagramms und des zweiten Skelettdiagramms in ein erstes integriertes Skelettdiagramm, wobei das erste integrierte Skelettdiagramm eine Vielzahl von Gelenkwinkeln zu dem t-ten Zeitpunkt umfasst, wobei die Gelenkwinkel einer Vielzahl von Gelenken des ersten menschlichen Körpers entsprechen; und
als Reaktion auf Bestimmen, dass das erste integrierte Skelettdiagramm eine spezifizierte Bedingung erfüllt, Erhalten einer Vielzahl von Winkelwerten der Gelenkwinkel und Verwenden der Winkelwerte als die Geh-Gliedmaßen-Merkmalswerte des Benutzers zu dem t-ten Zeitpunkt; und
Auswerten (S540) eines Gangs des Benutzers (199) basierend auf den Schrittmerkmalwerten und der Geh-Gliedmaßen-Merkmalswerte.

2. Gangbewertungsvorrichtung (110) gemäß Anspruch 1, wobei c:
als Reaktion auf Bestimmen, dass Y der Schrittmerkmalswerte und der Geh-Gliedmaßen-Merkmalswerte des Benutzers (199) einen entsprechenden ersten statistischen Standard nicht erfüllen, Bestimmen, dass der Gang des Benutzers (199) zu einem abnormalen Gang gehört, wobei Y eine bestimmte Zahl ist.

3. Gangbewertungsvorrichtung (110) gemäß Anspruch 2, wobei der Benutzer (199) zu einer spezifischen Gruppe gehört, und das Verfahren umfasst:
Erhalten einer Vielzahl von Referenz-Schritt-Merkmalswerten und einer Vielzahl von Referenz-Geh-Gliedmaßen-Merkmalswerten einer Vielzahl von Gruppenmitgliedern der spezifischen Gruppe, und dementsprechend Schätzen des ersten statistischen Standards von jedem der Schritt-Merkmalswerte und jedem der Geh-Gliedmaßen-Merkmalswerte.

4. Gangbewertungsvorrichtung (110) gemäß Anspruch 2, wobei der Prozessor weiterhin durchführt:
Erhalten einer Vielzahl von historischen Schrittmerkmalswerten und einer Vielzahl von historischen Geh-Gliedmaßen-Merkmalswerte des Benutzers (199), wobei die historischen Schrittmerkmalswerte und die historischen Geh-Gliedmaßen-Merkmalswerte den Schrittmerkmalswerten und den Geh-Gliedmaßen-Merkmalswerte des Benutzers (199) entsprechen; und
Bestimmen, basierend auf einem spezifischen Verhältnis jedes der historischen Schrittmerkmalwerte und jedes der historischen Geh-Gliedmaßen-Merkmalswerte, des ersten statistischen Standards jedes der Schrittmerkmalwerte und jedes der Geh-Gliedmaßen-Merkmalswerte.

5. Gangbewertungsvorrichtung (110) gemäß Anspruch 1, wobei der Prozessor durchführt:
Auswählen einer Anzahl N spezifischer Werte aus den Schrittmerkmalswerten und den Geh-Gliedmaßen-Merkmalswerte und Abbilden der spezifischen Werte in eine Vielzahl von Abbildungswerten gemäß einer Anzahl K von Referenzbasen, die den spezifischen Werten entsprechen, wobei N und K positive ganze Zahlen sind und jeder der Abbildungswerte in einen vorgegebenen Bereich fällt;
Durchführen einer Gewichtungsoperation an den Abbildungswerten, um ein Gewichtungsoperationsergebnis zu erhalten; und
als Reaktion auf Bestimmen, dass das Ergebnis der Gewichtungsoperation einen zweiten statistischen Standard nicht erfüllt, die Bestimmen, dass der Gang des Benutzers (199) zu einem abnormalen Gang gehört.

6. Gangbewertungsvorrichtung (110) gemäß Anspruch 5, wobei die spezifischen Werte einen ersten spezifischen Wert umfassen und der Prozessor
durchführt:
Erhalten eines Referenzmittelwerts und eines Referenzdifferenzfaktors, die dem ersten spezifischen Wert entsprechen, und entsprechendes Schätzen der Referenzbasen, die dem ersten spezifischen Wert entsprechen.

7. Gangbewertungsvorrichtung (110) gemäß Anspruch 6, wobei der Benutzer (199) zu einer spezifischen Gruppe gehört, die spezifische Gruppe eine Vielzahl von Gruppenmitgliedern umfasst und jedes der Gruppenmitglieder eine Vielzahl von Referenzschritt-Merkmalswerten und eine Vielzahl von Referenzschritt-Merkmalswerten aufweist, und der Prozessor durchführt:
Ermitteln eines ersten Referenzwertes, der dem ersten spezifischen Wert entspricht, aus den Referenzschritt-Merkmalswerten und den Referenz-Gehgliedmaßen-Merkmalswerten jedes der Gruppenmitglieder; und
Erhalten eines Mittelwerts und einer Standardabweichung des ersten Referenzwerts für jedes der Gruppenmitglieder und Definieren des Mittelwerts und der Standardabweichung als Referenzmittelwert bzw. Referenzdifferenzfaktor des ersten spezifischen Werts.

8. Gangbewertungsvorrichtung (110) gemäß Anspruch 6, wobei die Abbildungswerte einen ersten Abbildungswert umfassen, der dem ersten spezifischen Wert entspricht, der Referenzmittelwert als M dargestellt wird, der Referenzdifferenzfaktor als S dargestellt wird und die Referenzbasen, die dem ersten spezifischen Wert entsprechen, als M+iS dargestellt werden, wobei i eine ganze Zahl ist, *i* ∈ [-*a*, ..., +*a*] und a eine positive ganze Zahl ist, und der Prozessor durchführt:
als Reaktion auf Bestimmen, dass der erste spezifische Wert zwischen einer j-ten Referenzbasis und einer j+1-ten Referenzbasis in den Referenzbasen liegt, Bestimmen, dass der erste Abbildungswert j+ 1 +b ist, wobei 1 ≤ *j* ≤ *K* - 1, und b eine Konstante ist;
als Reaktion auf Bestimmen, dass der erste spezifische Wert kleiner als eine erste Referenzbasis in den Referenzbasen ist, Bestimmen, dass der erste Abbildungswert 1+b ist; und
als Reaktion auf Bestimmen, dass der erste spezifische Wert größer ist als eine K-te Referenzbasis in den Referenzbasen, Bestimmen, dass der erste Abbildungswert K + 1 +b ist.

9. Gangbewertungsvorrichtung (110) gemäß Anspruch 5, wobei der Benutzer (199) zu einer spezifischen Gruppe gehört, die spezifische Gruppe eine Vielzahl von Gruppenmitgliedern umfasst und jedes der Gruppenmitglieder eine Vielzahl von Referenzschritt-Merkmalswerten und eine Vielzahl von Referenzschritt-Merkmalswerten aufweist, und der Prozessor durchführt:
Erhalten einer Anzahl N von Referenzwerten, die den spezifischen Werten entsprechen, aus den Referenzschritt-Merkmalswerten und den Referenz- Geh-Gliedmaßen-Merkmalswerte jedes der Gruppenmitglieder;
Abbilden der Referenzwerte jedes der Gruppenmitglieder gemäß den Referenzbasen, die jedem der spezifischen Werte entsprechen, in eine Vielzahl von Referenzabbildungswerten, wobei jeder der Referenzabbildungswerte in den vorbestimmten Bereich fällt,
Durchführen der Gewichtungsoperation an den Referenzabbildungswerten jedes der Gruppenmitglieder, um ein Referenzgewichtungs-Operationsergebnis für jedes der Gruppenmitglieder zu erzeugen; und
Bestimmen des zweiten statistischen Standards basierend auf dem Ergebnis der Referenzgewichtungsoperation für jedes der Gruppenmitglieder.

10. Gangbewertungsvorrichtung (110) gemäß Anspruch 2 oder 5, wobei die Schrittmerkmalswerte und die Geh-Gliedmaßen-Merkmalswerte eine Schritt-zu-Schritt-Variation umfassen, und der Prozessor durchführt:
als Reaktion auf Bestimmen, dass der Gang des Benutzers (199) zu dem abnormalen Gang gehört und die Schritt-zu-Schritt-Variation einen dritten statistischen Standard erfüllt, Bestimmen, dass der Gang des Benutzers (199) zu einem neuropathischen Gang gehört; und
als Reaktion auf Bestimmen, dass der Gang des Benutzers (199) nicht zu dem abnormalen Gang gehört oder die Schritt-zu-Schritt-Abweichung nicht dem dritten statistischen Standard entspricht, Bestimmen, dass der Gang des Benutzers (199) zu einem nicht-neuropathischen Gang gehört.

11. Gangbewertungsvorrichtung (110) gemäß Anspruch 10, wobei der Benutzer (199) zu einer spezifischen Gruppe gehört, die spezifische Gruppe eine Vielzahl von Gruppenmitgliedern umfasst und jedes der Gruppenmitglieder die entsprechende Schritt-zu-Schritt-Variation aufweist, und der Prozessor durchführt:
Bestimmen, basierend auf der Schritt-zu-Schritt-Variation jedes der Gruppenmitglieder, des dritten statistischen Standards.

12. Gangbewertungsvorrichtung (110) gemäß Anspruch 10, wobei als Reaktion auf Bestimmen, dass der Gang des Benutzers (199) zu dem nicht-neuropathischen Gang oder dem neuropathischen Gang gehört, ein Freigabevorschlag bereitgestellt wird.

13. Gangbewertungsvorrichtung (110) gemäß Anspruch 12, wobei als Reaktion auf Bestimmen, dass der Gang des Benutzers (199) zu dem nicht-neuropathischen Gang gehört, ein Krafttrainingsvorschlag, der dem nicht-neuropathischen Gang entspricht, als der Freigabevorschlag bereitgestellt wird.

14. Gangbewertungsvorrichtung (110) gemäß Anspruch 12, wobei als Reaktion auf Bestimmen, dass der Gang des Benutzers (199) zu dem neuropathischen Gang gehört, ein rhythmischer Gangtrainingsvorschlag, der dem neuropathischen Gang entspricht, als der Freigabevorschlag bereitgestellt wird.

15. Gangbewertungssystem (100), das umfasst:
eine Druckerfassungsvorrichtung (120);
mindestens eine Gliedmaßen-Erfassungsvorrichtung (131-13Z); und
eine Gangbewertungsvorrichtung (110), die konfiguriert ist, um:
von der Druckerfassungsvorrichtung (120) eine Vielzahl von Druckwerten (PV) eines Benutzers (199) zu erhalten, der auf der Druckerfassungsvorrichtung (120) geht, wobei die Druckwerte (PV) einer Vielzahl von Schritten des Benutzers (199) entsprechen;
eine Vielzahl von Schrittmerkmalswerten des Benutzers (199) basierend auf den Druckwerten (PV) zu erhalten;
eine Vielzahl von Geh-Gliedmaßen-Merkmalswerten zu erhalten, wenn der Benutzer (199) auf der Druck-Erfassungsvorrichtung (120) geht, basierend auf Erfassungsdaten, die von der mindestens einen Gliedmaßen-Erfassungsvorrichtung (131-13Z) bereitgestellt werden, wobei die mindestens eine Gliedmaßen-Erfassungsvorrichtung mindestens eine erste Videokamera und eine zweite Videokamera mit unterschiedlichen Bildbereichen umfasst, und wobei die Gangbewertungsvorrichtung (110) angepasst ist, durchzuführen:
Erhalten, zu einem t-ten Zeitpunkt, eines ersten Gehbildes, das von der ersten Videokamera aufgenommen wird, wenn der Benutzer auf der Druckerkennungsvorrichtung geht, und Erhalten eines ersten Skelettdiagramms in dem ersten Gehbild,
Erhalten zu einem t-ten Zeitpunkt eines zweiten Gehbildes, das von der zweiten Videokamera aufgenommen wurde, wenn der Benutzer auf der Druckerfassungsvorrichtung geht, und Erhalten eines zweiten Skelettdiagramms in dem zweiten Gehbild, wobei das erste Skelettdiagramm und das zweite Skelettdiagramm einem ersten menschlichen Körper entsprechen;
Projizieren, basierend auf einer relativen Position zwischen der ersten Videokamera und der zweiten Videokamera, des ersten Skelettdiagramms und des zweiten Skelettdiagramms in ein erstes integriertes Skelettdiagramm, wobei das erste integrierte Skelettdiagramm eine Vielzahl von Gelenkwinkeln zu dem t-ten Zeitpunkt umfasst, wobei die Gelenkwinkel einer Vielzahl von Gelenken des ersten menschlichen Körpers entsprechen; und
als Reaktion auf Bestimmen, dass das erste integrierte Skelettdiagramm eine spezifizierte Bedingung erfüllt, Erhalten einer Vielzahl von Winkelwerten der Gelenkwinkel und Verwenden der Winkelwerte als die Geh-Gliedmaßen-Merkmalswerte des Benutzers zu dem t-ten Zeitpunkt; und
Bewerten eines Gangs des Benutzers (199) basierend auf den Schrittmerkmalswerten und der Geh-Gliedmaßen-Merkmalswerte.

16. System gemäß Anspruch 15, wobei die Druckerkennungsvorrichtung (120) eine Druckerkennungseinlage umfasst, die an einem Fuß des Benutzers (199) getragen wird, wobei die Druckerkennungseinlage die Druckwerte (PV) der Schritte des Benutzers (199) erkennt; oder
die Druckerkennungsvorrichtung (120) eine Druckerkennungsmatte umfasst, die mit einer Vielzahl von Druckdetektoren (120a, 120b) verteilt ist, wobei die Druckerkennungsmatte die Druckwerte (PV) der Schritte des Benutzers (199) durch die Druckdetektoren erkennt.

17. System gemäß Anspruch 15, wobei die mindestens eine Gliedmaßen-Erfassungsvorrichtung (131-13Z) eine Vielzahl von dynamischen Erfassungselementen umfasst, die an dem Benutzer getragen werden.

## Revendications

1. Un dispositif (110) d'évaluation d'une démarche, comprenant :
un circuit de stockage, stockant un code de programmation ;
un processeur, relié au circuit de stockage et accédant au code de programmation pour mettre en oeuvre :
le fait (S510) d'obtenir, à partir d'un dispositif (120) de détection de pression, une pluralité de valeurs de pression (PV) d'un utilisateur (199) marchant sur le dispositif (120) de détection de pression, les valeurs de pression (PV) correspondant à une pluralité de pas de l'utilisateur (199) ;
le fait (S520) d'obtenir une pluralité de valeurs de caractéristiques de pas de l'utilisateur (199) sur la base des valeurs de pression (PV) ;
le fait (S530) d'obtenir une pluralité de valeurs de caractéristiques de membre en cours de marche lorsque l'utilisateur (199) marche sur le dispositif (120) de détection de pression sur la base de données de détection fournies par au moins un dispositif (131-13Z) de détection de membre,
dans lequel
ledit au moins un dispositif de détection de membre comprend au moins une première caméra vidéo et une deuxième caméra vidéo ayant des plages d'imagerie différentes, et le processeur est adapté pour mettre en oeuvre :
le fait d'obtenir, à un t-ième instant, une première image de marche capturée par la première caméra vidéo lorsque l'utilisateur marche sur le dispositif de détection de pression, et le fait d'obtenir un premier diagramme de squelette dans la première image de marche ;
le fait d'obtenir, au t-ième instant, une deuxième image de marche capturée par la deuxième caméra vidéo lorsque l'utilisateur marche sur le dispositif de détection de pression, et le fait d'obtenir un deuxième diagramme de squelette dans la deuxième image de marche, le premier diagramme de squelette et le deuxième diagramme de squelette correspondant à un premier corps humain ;
le fait de projeter, sur la base d'une position relative entre la première caméra vidéo et la deuxième caméra vidéo, le premier schéma de squelette et le deuxième schéma de squelette dans un premier schéma de squelette intégré, le premier schéma de squelette intégré comprenant une pluralité d'angles d'articulation au t-ième instant, les angles d'articulation correspondant à une pluralité d'articulations du premier corps humain ; et
en réponse à la détermination que le premier schéma de squelette intégré satisfait une condition spécifiée, le fait d'obtenir une pluralité de valeurs d'angle des angles d'articulation, et le fait de prendre les valeurs d'angle comme valeurs de caractéristiques des membres de l'utilisateur en cours de marche au t-ième instant ; et
le fait (S540) d'évaluer une démarche de l'utilisateur (199) sur la base des valeurs de caractéristiques de pas et des valeurs de caractéristiques de membres en cours de marche.

2. Le dispositif (110) d'évaluation de démarche tel que décrit dans la revendication 1, dans lequel, c :
en réponse à la détermination que Y des valeurs de caractéristiques de pas et des valeurs de caractéristiques de membres en cours de marche de l'utilisateur (199) ne satisfont pas à une première norme statistique correspondante, le fait de déterminer que la démarche de l'utilisateur (199) appartient à une démarche anormale, Y étant un nombre spécifié.

3. Le dispositif (110) d'évaluation de démarche tel que décrit dans la revendication 2, dans lequel l'utilisateur (199) appartient à un groupe spécifique, et le procédé comprend :
le fait d'obtenir une pluralité de valeurs de référence de caractéristiques de pas et une pluralité de valeurs de référence de caractéristiques de membre en cours de marche d'une pluralité de membres du groupe spécifique, et d'estimer en conséquence la première norme statistique de chacune des valeurs de caractéristiques de pas et de chacune des valeurs de caractéristiques de membre en cours de marche.

4. Le dispositif (110) d'évaluation de démarche tel que décrit dans la revendication 2, dans lequel le processeur met en outre en oeuvre :
le fait d'obtenir une pluralité de valeurs historiques de caractéristiques de pas et une pluralité de valeurs historiques de caractéristiques de membre en cours de marche de l'utilisateur (199), les valeurs historiques de caractéristiques de pas et les valeurs historiques de caractéristiques de membre en cours de marche correspondant aux valeurs de caractéristiques de pas et aux valeurs de caractéristiques de membre en cours de marche de l'utilisateur (199) ; et
le fait de déterminer, sur la base d'un rapport spécifique de chacune des valeurs historiques de caractéristiques de pas et de chacune des valeurs historiques de caractéristiques de membre en cours de marche, la première norme statistique de chacune des valeurs de caractéristiques de pas et de chacune des valeurs de caractéristiques de membre en cours de marche.

5. Le dispositif (110) d'évaluation de démarche tel que décrit dans la revendication 1, dans lequel le processeur met en oeuvre :
le fait de sélectionner un nombre N de valeurs spécifiques parmi les valeurs de caractéristiques de pas et les valeurs de caractéristiques de membre en cours de marche, et de mapper les valeurs spécifiques dans une pluralité de valeurs de mappage selon un nombre K de bases de référence correspondant aux valeurs spécifiques, N et K étant des entiers positifs, et chacune des valeurs de mappage se situant dans une gamme prédéterminée ;
le fait d'effectuer une opération de pondération sur les valeurs de mappage pour obtenir un résultat d'opération de pondération ; et
en réponse à la détermination que le résultat de l'opération de pondération ne satisfait pas à une deuxième norme statistique, le fait de déterminer que la démarche de l'utilisateur (199) appartient à une démarche anormale.

6. Le dispositif (110) d'évaluation de démarche tel que décrit dans la revendication 5, dans lequel les valeurs spécifiques comprennent une première valeur spécifique, et le processeur met en oeuvre :
le fait d'obtenir une moyenne de référence et un facteur de différence de référence correspondant à la première valeur spécifique, et d'estimer en conséquence les bases de référence correspondant à la première valeur spécifique,

7. Le dispositif (110) d'évaluation de démarche tel que décrit dans la revendication 6, dans lequel l'utilisateur (199) appartient à un groupe spécifique, le groupe spécifique comprend une pluralité de membres du groupe, et chacun des membres du groupe a une pluralité de valeurs de référence de caractéristiques de pas et une pluralité de valeurs de référence de caractéristiques de membre en cours de marche, et le processeur met en oeuvre :
le fait de trouver une première valeur de référence correspondant à la première valeur spécifique à partir des valeurs de référence de caractéristiques de pas et des valeurs de référence de caractéristiques de membre en cours de marche de chacun des membres du groupe ; et
le fait d'obtenir une moyenne et un écart type de la première valeur de référence de chacun des membres du groupe, et de définir respectivement la moyenne et l'écart type comme étant la moyenne de référence et le facteur de différence de référence de la première valeur spécifique,

8. Le dispositif (110) d'évaluation de démarche tel que décrit dans la revendication 6, dans lequel les valeurs mappées comprennent une première valeur mappée correspondant à la première valeur spécifique, la moyenne de référence est représentée par M, le facteur de différence de référence est représenté par S, et les bases de référence qui correspondent à la première valeur spécifique sont représentées par M+iS, i étant un entier, i E [-a,.. .,+a], et a étant un entier positif, et le processeur met en oeuvre :
en réponse à la détermination que la première valeur spécifique est comprise entre une j-ième base de référence et une j+1-ième base de référence dans les bases de référence, le fait de déterminer que la première valeur mappée est j+1+b, dans lequel 1 ≤ *j* ≤ *K*-1, et b est une constante ;
en réponse à la détermination que la première valeur spécifique est inférieure à une première base de référence dans les bases de référence, le fait de déterminer que la première valeur mappée est 1+b ; et
en réponse à la détermination que la première valeur spécifique est supérieure à une K-ième base de référence dans les bases de référence, le fait de déterminer que la première valeur de mappage est K+1+b.

9. Le dispositif (110) d'évaluation de démarche tel que décrit dans la revendication 5, dans lequel l'utilisateur (199) appartient à un groupe spécifique, le groupe spécifique comprend une pluralité de membres du groupe, et chacun des membres du groupe a une pluralité de valeurs de référence de caractéristiques de pas et une pluralité de valeurs de référence de caractéristiques de membre en cours de marche, et le processeur met en oeuvre :
le fait d'obtenir un nombre N de valeurs de référence correspondant aux valeurs spécifiques à partir des valeurs de référence de caractéristiques de pas et des valeurs de référence de caractéristiques de marche de chacun des membres du groupe ;
le fait de mapper, selon les bases de référence correspondant à chacune des valeurs spécifiques, les valeurs de référence de chacun des membres du groupe en une pluralité de valeurs de mappage de référence, chacune des valeurs de mappage de référence se situant dans la plage prédéterminée,
le fait d'effectuer l'opération de pondération sur les valeurs de mappage de référence de chacun des membres du groupe pour générer un résultat d'opération de pondération de référence de chacun des membres du groupe ; et
le fait de déterminer, sur la base du résultat de l'opération de pondération de référence de chacun des membres du groupe, la deuxième norme statistique.

10. Le dispositif (110) d'évaluation de démarche tel que décrit dans la revendication 2 ou la revendication 5, dans lequel les valeurs de caractéristiques de pas et les valeurs de caractéristiques de membre en cours de marche comprennent une variation de foulée à foulée, et le processeur met en oeuvre :
en réponse à la détermination que la démarche de l'utilisateur (199) appartient à la démarche anormale, et que la variation de foulée à foulée satisfait une troisième norme statistique, le fait de déterminer que la démarche de l'utilisateur (199) appartient à une démarche neuropathique ; et
en réponse à la détermination que la démarche de l'utilisateur (199) n'appartient pas à la démarche anormale, ou que la variation de foulée à foulée ne satisfait pas la troisième norme statistique, le fait de déterminer que la démarche de l'utilisateur (199) appartient à une démarche non neuropathique,

11. Le dispositif (110) d'évaluation de démarche tel que décrit dans la revendication 10, dans lequel l'utilisateur (199) appartient à un groupe spécifique, le groupe spécifique comprend une pluralité de membres du groupe, et chacun des membres du groupe présente la variation de foulée à foulée correspondante, et le processeur met en oeuvre :
le fait de déterminer, sur la base de la variation de foulée à foulée de chacun des membres du groupe, la troisième norme statistique.

12. Le dispositif (110) d'évaluation de démarche tel que décrit dans la revendication 10, dans lequel en réponse à la détermination que la démarche de l'utilisateur (199) appartient à la démarche non neuropathique ou à la démarche neuropathique, une suggestion d'activation est fournie.

13. Le dispositif (110) d'évaluation de démarche tel que décrit dans la revendication 12, dans lequel en réponse à la détermination que la démarche de l'utilisateur (199) appartient à la démarche non neuropathique, une suggestion d'entraînement en force correspondant à la démarche non neuropathique est fournie en tant que suggestion d'activation.

14. Le dispositif (110) d'évaluation de démarche tel que décrit dans la revendication 12, dans lequel, en réponse à la détermination que la démarche de l'utilisateur (199) appartient à une démarche neuropathique, une suggestion d'entraînement à la marche rythmique correspondant à la marche neuropathique est fournie en tant que suggestion d'activation.

15. Un système (100) d'évaluation d'une démarche, comprenant :
un dispositif (120) de détection de pression ;
au moins un dispositif (131-13Z) de détection de membre ; et
un dispositif (110) d'évaluation d'une démarche, configuré pour :
obtenir, à partir du dispositif (120) de détection de pression, une pluralité de valeurs de pression (PV) d'un utilisateur (199) marchant sur le dispositif (120) de détection de pression, les valeurs de pression (PV) correspondant à une pluralité de pas de l'utilisateur (199) ;
obtenir une pluralité de valeurs de caractéristiques de pas de l'utilisateur (199) sur la base des valeurs de pression (PV) ;
obtenir une pluralité de valeurs de caractéristiques de membre en cours de marche lorsque l'utilisateur (199) marche sur le dispositif (120) de détection de pression sur la base de données de détection fournies par ledit au moins un dispositif de détection de membre (131-13Z),
dans lequel
ledit au moins un dispositif de détection de membre comprend au moins une première caméra vidéo et une deuxième caméra vidéo ayant des plages d'imagerie différentes, et
dans lequel
le dispositif (110) d'évaluation de démarche est adapté pour mettre en oeuvre :
le fait d'obtenir, à un t-ième instant, une première image de marche capturée par la première caméra vidéo lorsque l'utilisateur marche sur le dispositif de détection de pression, et d'obtenir un premier diagramme de squelette dans la première image de marche ;
le fait d'obtenir, au t-ième instant, une deuxième image de marche capturée par la deuxième caméra vidéo lorsque l'utilisateur marche sur le dispositif de détection de pression, et d'obtenir un deuxième diagramme de squelette dans la deuxième image de marche, le premier diagramme de squelette et le deuxième diagramme de squelette correspondant à un premier corps humain ;
le fait de projeter, sur la base d'une position relative entre la première caméra vidéo et la deuxième caméra vidéo, le premier schéma de squelette et le deuxième schéma de squelette dans un premier schéma de squelette intégré, le premier schéma de squelette intégré comprenant une pluralité d'angles d'articulation au t-ième instant, les angles d'articulation correspondant à une pluralité d'articulations du premier corps humain ; et
en réponse à la détermination que le premier schéma de squelette intégré satisfait une condition spécifiée, le fait d'obtenir une pluralité de valeurs d'angle des angles d'articulation, et de prendre les valeurs d'angle comme valeurs de caractéristiques des membres en cours de marches de l'utilisateur au t-ième instant ; et
le fait d'évaluer une démarche de l'utilisateur (199) sur la base des valeurs de caractéristiques de pas et des valeurs de caractéristiques des membres en cours de marches.

16. Le système tel que décrit dans la revendication 15, dans lequel le dispositif (120) de détection de pression comprend une semelle intérieure de détection de pression portée sur un pied de l'utilisateur (199), la semelle intérieure de détection de pression détectant les valeurs de pression (PV) des pas de l'utilisateur (199) ; ou
le dispositif (120) de détection de pression comprend un tapis de détection de pression réparti avec une pluralité de détecteurs de pression (120a, 120b), le tapis de détection de pression détectant les valeurs de pression (PV) des pas de l'utilisateur (199) par l'intermédiaire des détecteurs de pression.

17. Le système tel que décrit dans la revendication 15, dans lequel ledit au moins un dispositif de détection de membre (131-13Z) comprend une pluralité d'éléments de capture dynamique portés sur l'utilisateur.
